Europäisches Patentamt

European Patent Office

Office européen des brevets

(19)

(11) Numéro de publication: **0 306 360**
**A1**

(12) **DEMANDE DE BREVET EUROPEEN**

(21) Numéro de dépôt: **88401907.6**

(22) Date de dépôt: **22.07.88**

(51) Int. Cl.4: **C 07 K 3/00**
C 07 K 15/00, A 61 K 39/00,
G 01 N 33/68, G 01 N 33/564

(30) Priorité: **26.08.87 FR 8711946**

(43) Date de publication de la demande:
**08.03.89 Bulletin 89/10**

(84) Etats contractants désignés:
**BE DE ES GB GR IT LU NL**

(71) Demandeur: **DIATECH S.A.**
**15, rue des Grands Prés**
**F-92000 Nanterre (FR)**

(72) Inventeur: **Goldstein, Israel**
**47, boulevard Barbès**
**F-75018 Paris (FR)**

**Marié, François Noel**
**25, rue des Cottages**
**F-78630 Orgeval (FR)**

(74) Mandataire: **Peuscet, Jacques et al**
**Cabinet Peuscet 68, rue d'Hauteville**
**F-75010 Paris (FR)**

Une requête en rectification revendication 9 a été présentée conformément à la règle 88 CBE. Il est statué sur cette requête au cours de la procedure engagée devant la division d'examen (Directives relatives à l'examen pratiqué à l'OEB, A-V, 2.2).

(54) Procédé de préparation de fractions antigéniques destinées à la détection d'anticorps révélateurs d'un risque cardio-vasculaire, fractions antigéniques ainsi obtenues et leur utilisation pour le diagnostic d'un risque cardio-vasculaire.

(57) L'invention concerne un procédé de préparation de fractions antigéniques destinées à la détection des anticorps, dont la présence en certaine quantité dans le sérum sanguin d'un sujet est corrolée avec le risque d'accident cardio-vasculaire. On broie un tissu artériel, on le délipide et on fait agir au moins un agent pris dans le groupe formé par une solution aqueuse d'un sel de métal alcalin ou alcalino-terreux à température ambiante, un acide à chaud, de l'urée, de la guanidine et des enzymes protéolytiques. L'invention concerne également les fractions antigéniques ainsi obtenues et leur utilisation pour le diagnostic d'un risque cardio-vasculaire par électrosynérèse ou par un test Elifa ou par un test Elisa.

FIG. UNIQUE

**Description**

## PROCEDE DE PREPARATION DE FRACTIONS ANTIGENIQUES DESTINEES A LA DETECTION D'ANTICORPS REVELATEURS D'UN RISQUE CARDIO-VASCULAIRE, FRACTIONS ANTIGENIQUES AINSI OBTENUES ET LEUR UTILISATION POUR LE DIAGNOSTIC D'UN RISQUE CARDIO-VASCULAIRE.

La présente invention concerne un procédé de préparation de fractions antigéniques destinées à la détection des auto-anticorps, dont la présence en certaine quantité dans le sérum sanguin d'un sujet est corrélée avec le risque d'accident cardio-vasculaire dudit sujet. L'invention concerne également les fractions antigéniques ainsi obtenues et l'utilisation de ces fractions antigéniques pour le diagnostic d'un risque cardio-vasculaire soit par électrosynérèse, soit par un test de type ELIFA, soit par un test de type ELISA.

On sait que les maladies cardio-vasculaires et, notamment, l'athérosclérose sont une des principales causes de mortalité dans les pays civilisés. Cette affection est ubiquitaire et elle est favorisée par toute une série de facteurs, parmi lesquels on peut citer des facteurs dynamiques comme l'hypertension artérielle, des facteurs métaboliques comme le diabète ou l'augmentation du taux des lipides dans le sang, des facteurs toxiques tels que l'abus d'alcool ou de tabac. L'athérosclérose se traduit par une rigidification des artères en raison de la formation de plaques obstruantes à l'intérieur même du tissu artériel. Ces formations de plaques entraînent une réduction de la section de passage des artères et une réduction de l'élasticité et de la contractibilité des fibres musculaires élastiques de la zone où la plaque est formée. Ce phénomène a déjà été largement étudié (voir notamment GOLDSTEIN, FONTALIRAN, MARIÉ, GAY, Pathologie Biologie, 1986, 34, n$^0$ 7, 841-846) ; on sait ainsi que ces plaques avec dépôts calciques se forment généralement dans la zone de liaison entre la couche interne (ou intima) et la couche moyenne (ou média) du tissu artériel ; l'intima et la média n'étant pratiquement pas irriguées sont nourries et oxygénées à partir du courant sanguin qui circule dans l'artère ou du courant sanguin qui traverse les vaisseaux de la couche externe (ou advantice) du tissu artériel. Le dépôt une fois formé se trouve donc dans une zone d'où il est pratiquement inextractible et il en résulte que la maladie ne peut pratiquement pas régresser.

En outre, on a démontré, dans l'état de la technique susmentionné, que les lésions dues à une athérosclérose créent une production d'anticorps. On sait que l'organisme ne produit pas d'anticorps contre ses propres tissus lorsqu'ils sont normaux ; mais si le tissu est lésé, il devient différent sur le plan biochimique et il constitue lui-même un néo-antigène .L'organisme produit donc des anticorps contre ces néo-antigènes et ces anticorps viennent se fixer sur les néo-antigènes des plaques d'athérosclérose. Il se forme donc des complexes (néo-antigènes/auto-anticorps) et ces complexes viennent grossir le dépôt athérosclérotique, de sorte que la maladie, quand elle a apparu sur un sujet, est auto-entretenue par le phénomène immunologique ci-dessus décrit. Cette maladie auto-immune a donc une tendance à l'auto-aggravation dès qu'elle est apparue, et c'est la raison pour laquelle il est extrêmement souhaitable de disposer de tests permettant de déterminer sur un sujet l'apparition de la composante immunologique de la maladie et donc le niveau du risque d'accident cardio-vasculaire correspondant.

On sait que les immuno-globulines sont des matières premières pour la fabrication d'anticorps spécialisés. Dans le document de l'état de la technique précédemment cité, on a démontré que, sur les sites lésés par des plaques athérosclérotiques, on trouvait des immunoglobulines et du complément ($C_3$). Selon la présente invention, on a donc imaginé de repérer dans le sérum sanguin d'un sujet à étudier les immunoglobines susceptibles d'avoir été générées par l'organisme du sujet pour venir se fixer sur des zones d'athérosclérose du système artériel du sujet. Pour ce faire, selon l'invention, on prépare des fractions antigéniques que l'on fait réagir in-vitro avec le sérum du sujet à étudier, le résultat de la réaction étant révélé de façon appropriée en détectant et, éventuellement en mesurant la quantité de complexe antigène-anticorps formé.

La présente invention a donc pour objet un procédé de préparation de fraction(s) antigénique(s) destinée(s) à la détection des auto-anticorps dont la présence en certaine quantité dans le sérum sanguin d'un sujet est corrélée avec le risque d'accident cardio-vasculaire dudit sujet, caractérisé par le fait que, dans une première phase, on réalise un broyat de tissu artériel humain constituant la matière première des fractions recherchées ; dans une deuxième phase, on délipide le broyat par traitement au(x) solvant(s) à température ambiante ; dans une troisième phase, on reprend le résidu solide de la deuxième phase et on fait agir sur ces constituants, pour obtenir une pluralité de fractions protéiniques, au moins un agent pris dans le groupe formé par une solution aqueuse d'un sel de métal alcalin ou alcalino-terreux à température ambiante, un acide à chaud, de l'urée, de la guanidine ou des enzymes protéolytiques.

Selon une première variante, au cours de la troisième phase susmentionnée, on soumet le résidu solide de la deuxième phase au traitement par solution(s) aqueuse(s) de sel de métal alcalin ou alcalino-terreux, on reprend le résidu de cette opération en le soumettant au traitement acide à chaud, puis on soumet tout ou partie du nouveau résidu ainsi obtenu à l'un des autres agents de traitement prévus. Après le traitement acide à chaud, on peut avantageusement fractionner en quatre parties le résidu non solubilisé, la première partie étant soumise à température ambiante à une solution aqueuse d'urée, la deuxième partie étant soumise à température ambiante à une solution aqueuse de guanidine, la troisième partie étant soumise à un traitement à l'élastase entre 30 et 45 °C et la quatrième partie étant soumise à un traitement à la pronase entre 30 et 45° C. De préférence, on réalise les traitements enzymatiques des troisième et quatrième parties du résidu à un pH compris entre 8,3 et 9,2 et on maintient lesdits traitements pendant un temps de 8 à 16 heures, ces traitements étant ensuite arrêtés par chauffage à 60-70° C pendant au moins 60 minutes. De préférence également, on soumet la première partie du résidu à une solution aqueuse d'urée à un pH compris entre 5,5 et 6,5, à une concentration comprise entre 7 et 9 moles/l, sous agitation, pendant 20 à 30 heures, et on soumet la deuxième partie du résidu à une solution aqueuse de guanidine à un pH compris entre 5,5 et 6,5, à une concentration comprise entre 3 et 5 moles/l, sous agitation, pendant 20 à 30 heures.

Selon une seconde variante, dans la troisième phase du procédé de préparation selon l'invention, on soumet le résidu solide de la deuxième phase au traitement par solution(s) aqueuse(s) de sel de métal alcalin ou alcalino-terreux, on reprend les fractions liquides obtenues pour reformer des fibres de collagène selon le procédé décrit dans la demande de brevet français 87-09297 et l'on traite lesdites fibres reconstituées par l'un des agents de traitement prévus pour ladite troisième phase. Avantageusement, on fractionne les fibres reconstituées en cinq parties, les quatre dernières parties étant soumises chacune à l'un des traitement pris dans le groupe formé par les traitements à l'urée, à la guanidine et aux enzymes précédemment définis, la première partie étant soumise à un traitement acide à chaud.

Dans l'une ou l'autre des variantes susmentionnées, le traitement acide à chaud est avantageusement réalisé au moyen d'une solution aqueuse d'acide trichloracétique à une concentration comprise entre 0,1 et 0,5 mole/1, à une tem pérature comprise entre 85 et 105°C, pendant un temps compris entre 15 et 45 minutes.

L'invention a également pour objet, à titre de produit industriel nouveau, les fractions antigéniques obtenues sous forme de solutions aqueuses dans le procédé ci-dessus défini.

La mise en oeuvre des fractions antigéniques ci-dessus définies pour la détection des anticorps dans le sérum du sujet à étudier peut être effectuée selon l'une ou l'autre de trois techniques, dont le principe est bien connu de l'homme de l'art : d'une part, l'électrosynérèse (voir notamment COONROD J.D. et RYTEL M.W., J. Lab. Clin. Med., 81, 770 (1973)), d'autre part, la technique ELIFA (voir notamment Journal of Immunological Methods, 77 (1985), 15-23) et enfin la technique ELISA (voir notamment ENGVALL et RUOSLAHTI, 1977, Int. J. Cancer, 20, 1), cette dernière permettant une évaluation quantitative, alors que les deux premières ne permettent qu'une évaluation qualitative du risque d'accident cardio-vasculaire.

La présente invention a donc également pour objet l'utilisation d'au moins une fraction antigénique telle que ci-dessus définie pour le diagnostic d'un risque cardio-vasculaire, caractérisée par le fait que l'on réalise une électrosynérèse en mettant en oeuvre, dans un support approprié, d'une part un liquide antigénique contenant au moins une desdites fractions antigéniques et, d'autre part, le sérum sanguin dudit sujet. Dans une première variante, le support de l'électrosynérèse est un gel d'agarose ; dans une deuxième variante, ce support est une bande d'acétate de cellulose, Avantageusement, la différence de potentiel entre les zones-réservoirs respectivement occupées par le liquide antigénique et le sérum est maintenue entre 100 et 200 volts pendant environ 60 à 90 minutes. Dans cette technique, les immunoglobulines du sérum à étudier migrent vers le pôle négatif alors que la plupart des autres constituants du sérum de même que les antigènes du liquide antigénique migrent vers le pôle positif ; les immunoglobulines rencontrent donc les antigènes, dont elles sont spécifiques, et se fixent sur ces antigènes pour former des complexes. Ces complexes antigènes/anticorps sont formés dans une zone plane ou cylindrique de recontre disposée entre les zones-réservoirs occupées par le liquide antigénique et le sérum.

Pour révéler la présence de ces complexes et obtenir l'indication qualitative recherchée, on peut opérer par coloration des complexes. Dans une première variante, le support est plongé dans un colorant ayant une affinité pour les protéines, puis dans un décolorant dont l'action ne laisse subsister que la trace colorée des complexes antigènes-anticorps ; le colorant utilisé est avantageusement le bleu de Coomasie. Dans un deuxième variante, le support est traité avec des immunoglobulines animales anti-immunoglobulines humaines (ci-après dénommées "anti-Ig"), ces anti-Ig étant marquées à la péroxydase de façon connue (voir notamment AVRAEMAS S., Int. Rev. Cytol., 1970, 27, 349-387 et ImmunoChemistry, 1976, 6, 43-49) ; la révélation des anti-Ig fixées par les anticorps du sérum, qui se sont complexés sur les antigènes du liquide antigénique pendant l'électrosynérèse, est assurée par un mélange (substrat précurseur de coloration-eau oxygénée) ; on peut utiliser, comme précurseur de coloration, la diaminobenzidine.

Selon la troisième technique, on peut utiliser dans un test ELISA, au moins une fraction antigénique selon l'invention, et on peut ainsi quantifier le diagnostic d'un risque cardio-vasculaire pour un sujet vivant. Selon cette technique, on dispose le liquide antigénique dans un récipient à surface préalablement traitée contenant une milieu tamponné ayant un pH compris entre 8,5 et 9,5 ; on laisse évaporer à sec et on lave le récipient ; on sature le récipient avec une solution aqueuse de sérum albumine d'origine animale tamponnée à un pH compris entre 7 et 7,6 pendant 15 à 45 minutes à température ambiante ; on dilue le sérum à étudier dans un tampon à pH compris entre 7 et 7,6 dans un rapport compris entre 1/50ème et 1/2000ème et on le verse dans le récipient en ajoutant du sérum albumine d'origine animale ; après un temps de contact de 30 à 60 minutes, on lave le récipient à l'eau physiologique et on ajoute des anti-Ig d'origine animale marquées, de façon connue, à la péroxydase ; après un temps de contact compris entre 30 et 60 minutes, on lave le récipient et on révèle les complexes (antigènes-anticorps-anti-Ig) formés par une solution aqueuse de précurseur de coloration (substrat) en présence d'eau oxygénée, la révélation étant stoppée après un temps donné chronométrée 4-5 s ; on lit l'intensité de la coloration obtenue en raison de la présence des complexes contenant les anti-Ig marquées et on en déduit l'évaluation chiffrée du risque cardio-vasculaire.

Avantageusement, la solution aqueuse de précurseur de colorant utilisée pour ce test ELISA est une solution d'orthophénylènediamine tamponnée à un pH compris entre 4,8 et 5,1 et l'arrêt de la révélation est obtenu par addition d'une solution aqueuse d'acide sulfurique et de métabisulfite de sodium.

Pour mieux faire comprendre l'objet de l'invention, on va décrire maintenant, à titre d'exemples purement

EP 0 306 360 A1

illustratifs et non limitatifs, deux modes de mise en oeuvre du procédé de préparation des fractions antigéniques en cause et trois modes de mise en oeuvre de l'utilisation de ces fractions antigéniques pour la détection des risques cardio-vasculaires d'un sujet.

On prépare les fractions antigéniques à partir d'un tissu artériel humain. On prend,par exemple, une aorte humaine et on sépare par dissection l'advantice pour ne conserver que l'intima et la média. On coupe cette matière première en lanières de 0,5 cm de large environ ; on procède alors à un lavage à l'eau en laissant cette matière première dans cent fois son poids d'eau pendant 24 heures sous légère agitation. On broie ensuite cette matière première dans un broyeur de type "Magimix 3500" pendant 15 minutes.

Le matériau obtenu est délipidé en le mettant tout d'abord dans 10 fois son volume d'éther éthylique pendant 24 heures sous faible agitation, puis en soumettant le résidu insoluble à 10 fois son volume de chloroforme pendant 24 heures sous faible agitation. La première extraction permet d'obtenir une fraction F1 ; la deuxième extraction permet d'obtenir une fraction F2.

Le résidu solide est ensuite soumis, après broyage, à une solution aqueuse comportant un tampon "PBS" constitué de phosphate mono- et disodique à concentration de 0,01 mole/l dans des proportions donnant un pH = 7,3, additionné de 9 °/oo de ClNa et contenant 1 mole/l de chlorure de sodium. Ce traitement est maintenu sous agitation à 4°C pendant 24 heures ; le résidu solide est ensuite séparé de la phase liquide et soumis, après broyage, à une solution aqueuse tamponnée avec le tampon PBS ci-dessus défini, ladite solution contenant, en outre, 2 moles/l de chlorure de sodium, le traitement s'effectuant sous agitation à 4°C pendant 24 heures ; la phase liquide est séparée et mélangée à la phase liquide précédemment séparée pour former une fraction F3. Le résidu solide est repris après broyage dans une solution aqueuse tamponnée (tampon PBS) de chlorure de calcium à 1 mole/l, cette extraction étant maintenue à 4°C sous forte agitation pendant 24 heures ; à la fin de l'extraction, on sépare la phase liquide et le résidu solide. On réalise ainsi successivement cinq extractions identiques avec une solution aqueuse molaire de chlorure de calcium. On mélange les cinq extraits liquides obtenus et on reprend le résidu solide dans une sixième extraction faite avec une solution aqueuse tamponnée (tampon PBS) de chlorure de calcium à 2 moles/l pendant 24 heures à 4°C sous forte agitation. On obtient ainsi un nouvel extrait liquide que l'on mélange aux cinq précédents pour obtenir une fraction F4. On mélange les fractions F3 et F4.

Les fractions F1 et F2 contiennent les lipoprotéines du tissu artériel traité. Les fractions F3 et F4 contiennent les protéines et les glycoprotéines hydrosolubles et en se référant à la demande de brevet français 87-09297, on constate que les fractions F3 et F4 peuvent être utilisées pour la fabrication de fibres de collagène reconstituées. A partir de cette constatation, on peut poursuivre le procédé de préparation selon deux variantes distinctes.

Dans la première variante, on reprend le résidu non solubilisé obtenu après la dernière extraction au chlorure de calcium et on le traite par 10 volumes d'acide trichloracétique 0,25N pour 1 volume de résidu ; ce traitement s'effectue à 96°C pendant 30 minutes sous agitation. On obtient ainsi une phase liquide F5 qui contient le collagène acido-soluble dénaturé et on isole le résidu non solubilisé que l'on sépare en quatre parties égales.

La première partie est alors traitée avec une soluble aqueuse à 8 moles/l d'urée ; la solution est à pH 6 ; le traitement est maintenu sous agitation pendant 24 heures et l'on obtient ainsi une phase liquide F6.

La deuxième partie du résidu solide est traitée par une solution aqueuse de guanidine à 4 moles/l ; la solution est à pH 6 ; le traitement est maintenu pendant 24 heures sous agitation ; on obtient ainsi une phase liquide F7.

La troisième partie du résidu est traitée par l'élastase pancréatique du porc, à raison de 1 mg pour 100 mg de résidu ; le traitement est réalisé à pH 8,8 dans un tampon TRIS-HCl (TRIS = Trihydroxyméthylaminométhane) à 37°C pendant 12 heures. L'arrêt de la réaction est obtenu en chauffant le milieu pendant 1 heure à 65°C. On obtient ainsi une phase liquide F8.

La quatrième partie du résidu solide est traitée avec de la pronase (enzyme de streptomyces griseum) à raison de 1 mg d'enzyme pour 100 mg de résidu. Le traitement est réalisé à pH 8,8 dans un tampon TRIS-HCl 0,1 mole pendant 12 heures à 37°C. L'arrêt de la réaction est obtenu en chauffant pendant 1 heure à 65°C. On obtient ainsi une phase liquide F9.

Dans la deuxième variante de ce procédé de fabrication, on reprend une partie des fractions F3/F4 qui contiennent toutes les protéines solubles de tissu artériel initial et l'on reconstitue à partir de cette phase liquide des fibres de collagène selon le procédé décrit dans la demande de brevet français 87-09297, dont le contenu est incorporé dans la présente demande à titre de référence. On reprend alors les fibres reconstituées ainsi obtenues et on les partage en cinq parties égales.

La première partie des fibres est soumise à l'action de l'acide trichloracétique à 96°C comme indiqué précédemment pour la première variante : on obtient ainsi une fraction liquide F'5. La deuxième partie des fibres est traitée par une solution aqueuse d'urée comme indiqué pour la première variante : on obtient ainsi une fraction liquide F'6. La troisième partie est traitée par une solution aqueuse de guanidine comme dans la première variante: on obtient ainsi une fraction liquide F'7. La quatrième partie des fibres est traitée à l'élastase comme indiqué dans la première variante : on obtient ainsi une fraction liquide F'8. La cinquième partie est traitée par la pronase comme dans la première variante : on obtient ainsi une fraction liquide F'9.

Lorsqu'on a ainsi obtenu les fractions liquides F1 à F9 ou F1 à F4 et F'5 à F'9, on dispose de fractions antigéniques susceptibles de mettre en évidence les autoanticorps générés par un sujet atteint d'athérosclérose. Les réactions des différents antigènes correspondant à ces différentes fractions sont

4

différentes pour le diagnostic et la méthode de diagnostic consistera donc à mettre en oeuvre une batterie de tests utilisant les différentes fractions antigéniques ci-dessus préparées.La recherche des anticorps envers les fractions F1 et F2 correspond à la recherche des complexes (antigène-anticorps) avec les dépôts lipidiques. La recherche des anticorps envers les fractions F3 et F4 correspond à la recherche des complexes (antigène-anticorps) envers les protéines solubles : glycoprotéines solubles, protéoglycane, procollagène, proélastine et surtout fibronectine. La recherche des anticorps envers la fraction F5 correspond à des complexes (antigène-anticorps) dus au collagène de structure (insoluble). La recherche des anticorps envers les fractions F6 et F7 correspond à la formation des complexes (antigène-anticorps) avec des glycoprotéines de structure. La recherche des anticorps envers les fractions F8 et F9 correspond à la formation de complexes (antigène-anticorps) avec des glycopeptides à base de collagène , d'élastine et de sucres (sucres aminés, acide neuraminique, et mannose, galactose, glucose.

L'utilisation de ces fractions antigéniques peut être effectuée selon trois méthodes différentes.

Dans la première méthode, on réalise une électrosynérèse, c'est-à-dire une électrophorèse à contre-courant selon la technique décrite par COONROD et RYTEL (document prédemment cité ). On réalise une lame de gel d'agarose dans laquelle on ménage deux cavités où l'on place respectivement l'une des fractions antigéniques précédemment obtenues et le sérum du sujet à étudier. On soumet la lame de gel d'agarose qui a une longueur de 60 mm à une différence de potentiel de 120 volts ; le courant est d'environ 2 milliampères. Le pH du gel d'agarose est maintenu à 8,8 par un tampon TRIS-glycine 0,1 molaire. Le pôle positif est placé du côté du sérum à étudier ; les immunoglobulines dudit sérum migrent vers le pôle négatif; les antigènes du liquide antigénique migrent vers le pôle positif. La mise sous tension provoque donc la rencontre des antigènes et des immunoglobulines spécifiques correspondantes sur une surface plane ou cylindrique positionnée entre les deux zones-réservoirs. Dans cette zone de rencontre, il se forme un complexe antigène-anticorps .

Après 70 minutes d'électrosynérèse, on plonge la lame de gel pendant 7 minutes dans une solution à 1,5 % en poids de bleu de Coomasie dans un solvant éthanol/acide acétique/eau dans la proportion volumique 4/1/5 ; le colorant colore préférentiellement les protéines, c'est-à-dire les complexes antigènes/anticorps formés. Après avoir lavé la lame de gel, on la plonge ensuite dans une solution décolorante acide acétique/éthanol/eau dans la proportion pondérale 1/4/5 pendant un temps de 7 minutes. On obtient ainsi une décoloration du gel, seuls restant colorés, les complexes formés dans la zone de rencontre. On obtient ainsi une détermination qualitative de la présence dans le sérum à étudier des anticorps caractérisant le risque d'accident cardio-vasculaire.

Selon une deuxième technique, on réalise l'électrosynérèse ci-dessus mentionnée sur une bande d'acétate de cellulose au lieu d'utiliser une bande de gel d'agarose. La bande d'acétate de cellulose peut être également révélée avec le bleu de Coomasie, mais on peut également utiliser la technique ELIFA décrite par PINON, THOANNES et GRUSON, Journal of Immunological Methods, 77 (1985), 15-23. Dans ce cas, on fait agir sur la bande-support des immunoglobulines d'origine animale anti-immunoglobulines humaines. Ces anti-immuno-globulines (anti-Ig) peuvent être spécifiques des immunoglobulines humaines de type G, M, A et E. Ces anti-Ig sont marquées à la péroxydase selon la technique d'AVRAEMAS (documents précédemment cités). Dans la zone de rencontre de la bande d'acétate de cellulose se sont formés des complexes antigènes/anticorps et l'anti-Ig utilisée se fixe sur l'anticorps correspondant du complexe. On obtient ainsi un complexe antigène/anticorps/anti-Ig dont on révèle la présence par réaction avec l'orthophénylènediamine en présence d'eau oxygénée. La coloration rouge définit l'existence dans le sérum à étudier des anticorps de type correspondant à l'anti-Ig utilisée.

Selon une troisième technique, on réalise un test permettant une évaluation quantitative des anticorps dans le sérum à étudier ; ce test correspondant à la technique ELISA. On utilise une plaque de polyvinyle traitée en surface et fournie par la société "NUNC", ladite plaque comportant une pluralité de godets. Dans un godet, on met en place 150 µl de tampon carbonate de sodium 0,05 molaire à pH 9 et 0,1-1 µg d'une quelconque des fractions antigéniques préparées comme ci-dessus indiqué. On laisse la plaque à 37° C pendant 12 heures et on laisse ensuite 72 heures à la température ambiante pour obtenir un séchage complet. On lave ensuite le godet ainsi préparé avec de l'eau physiologique contenant 2°/oo du détergent vendu sous la dénomination commerciale "TWEEN 20" (mélange d'ester laurique de sorbitol et d'anhydride du sorbibol, principalement monolaurate de sorbitan oxyéthyléné à 20 moles d'oxyde d'éthylène). Cette opération de lavage est réalisée par trois lavages successifs de 5 minutes chacun. On ajoute ensuite dans le godet 200 µl de sérum-albumine d'origine bovine à une concentration de 1% en volume dans un tampon "PBS", le pH étant ainsi maintenu à 7,3. Le sérum-albumine est laissé en contact avec le godet pendant 30 minutes à la température ambiante après quoi le godet est vidé : cette saturation au sérum-albumine permet d'occuper dans le godet les sites non occupés par l'antigène.

Le sérum humain à étudier est préparé en dilution à 1% en poids dans un tampon "PBS", ce tampon étant additionné de 1% en volume de sérum-albumine d'origine bovine et de 2°/oo en volume de détergent vendu sous la dénomination com merciale "TWEEN 20". On met en place dans le godet considéré 100 µl du sérum dilué comme ci-dessus indiqué et on le laisse dans le godet pendant 30 minutes à la température ambiante. Les anticorps du sérum ne peuvent se fixer que sur les sites occupés par les antigènes, les autres sites étant bloqués par le sérum albumine.

Après avoir vidé le godet, on le lave par cinq lavages successifs avec de l'eau physiologique additionnée de 2 °/oo en volume de détergent "TWEEN 20". On ajoute alors dans le godet 100 µl d'immunoglobulines de lapin anti-immunoglobulines humaines (dite "anti-Ig"). Ces anti-Ig sont marquées à la peroxydase de façon connue ; elles sont utilisées à la concentration de 1/5 000 en dilution dans le même tampon "PBS" que celui utilisé pour la dilution du sérum humain. On laisse les anti-Ig dans le godet pendant 30 minutes à la température ambiante. On vide ensuite le godet et on le lave par cinq lavages successifs comme avant la mise en place des anti-Ig.

Au cours des manipulations précédentes, on a fixé sur les parois du godet l'antigène correspondant à la fraction antigénique utilisée et on a ensuite amené par le sérum à étudier l'anticorps spécifique dudit antigène. On a donc formé le complexe antigènes/anticorps ; après quoi, on a amené les anti-Ig correspondant aux anticorps du complexe. On a donc formé un complexe (antigènes/anticorps/anti-Ig) et ce complexe est marqué à la péroxydase, puisque l'anti-Ig fixée était marquée à la péroxydase. Il reste donc à révéler ce complexe marqué à la péroxydase.

Pour ce faire, on prépare une solution d'orthophénylènediamine à 4 mg/ml dans un tampon à 0,1 mole/l de citrate de sodium (pH = 5) et on y ajoute de l'eau oxygénée à 110 volumes à raison de 2 µl/ml de tampon. On met en place 100 µl de cette solution dans le godet qui a servi de récipient de réaction. Une coloration brune apparaît. La réaction est stoppée après 6 minutes en ajoutant dans le godet 100 µl d'acide sulfurique 2N pour augmenter le pH additionné de 2% en poids de métabisulfite de sodium pour faire disparaître toute trace d'oxydant.

Le contenu du godet ainsi coloré est présenté en lecture sur un lecteur optique à deux faisceaux fonctionnant à 490 nanomètres ; le lecteur est du type "M 700" vendu par la société "DYNATECH". On note les valeurs de l'intensité colorimétrique après soustraction de la valeur correspondant à la coloration du fond. Pour le lecteur susmentionné, les valeurs comprises entre 0,2 et 2 sont considérées comme pathologiques de façon proportionnelle.

Dans cette technique, les plaques de polyvinyle comportent généralement un assez grand nombre de godets de façon à pouvoir effectuer les tests avec toute la gamme des fractions antigéniques dont la préparation a été ci-dessus indiquée et avec des anti-Ig spécifiques des immunoglobulines humaines de type G, A, M et E.

Les essais réalisés concernant les différentes fractions antigéniques F1 à F9 ou F1 à F4 et F'5 à F'9 ont démontré, en utilisant pour les tests ELISA des anti-Ig de type G, M, A et E, que l'on obtenait, selon le couplage (fraction antigénique/anti-Ig de type donné), des caractéristiques de diagnostic différentes. A titre d'exemples, on va donner ci-après les résultats obtenus en utilisant les fractions antigéniques F8 et F9 et des anti-Ig de type G ou M. Dans le tableau ci-après, les chiffres fournis sont les densités optiques lues sur le lecteur optique précédemment mentionné.

Le groupe de malades comportait 29 patients ayant un âge moyen de 60 ans. Le groupe des témoins jeunes comportait 44 sujets ayant un âge moyen de 20 ans. Le groupe des témoins âgés comportait 30 sujets ayant un âge moyen de 64 ans.

| | | sujets-témoins jeunes | sujets-témoins âgés | malades |
|---|---|---|---|---|
| nombre | | 44 | 30 | 29 |
| Fraction antigénique F8 et anti-Ig de type G | moyenne | 0,06 ± 0,09 | 0,08 ± 0,06 | 0,71 ± 0,54 |
| | médiane | 0,04 | 0,06 | 0,46 |
| Fraction antigénique F9 et anti-Ig de type G | moyenne | 0,37 ± 0,43 | 0,36 ± 0,26 | 0,84 ± 0,39 |
| | médiane | 0,02 | 0,03 | 0,83 |
| Fraction antigénique F8 et anti-Ig de type M | moyenne | 0,08 ± 0,09 | 0,14 ± 0,14 | 0,49 ± 0,44 |
| | médiane | 0,06 | 0,095 | 0,33 |
| Fraction antigénique F9 et anti-Ig de type M | moyenne | 0,18 ± 0,25 | 0,16 ± 0,12 | 0,7 · ± 0,39 |
| | médiane | 0,08 | 0,12 | 0,65 |

EP 0 306 360 A1

On constate qu'il n'y a que peu de différence entre les témoins jeunes et âgés, mais que, par contre, il y a une différence importante entre les groupes-témoins et le groupe de malades.

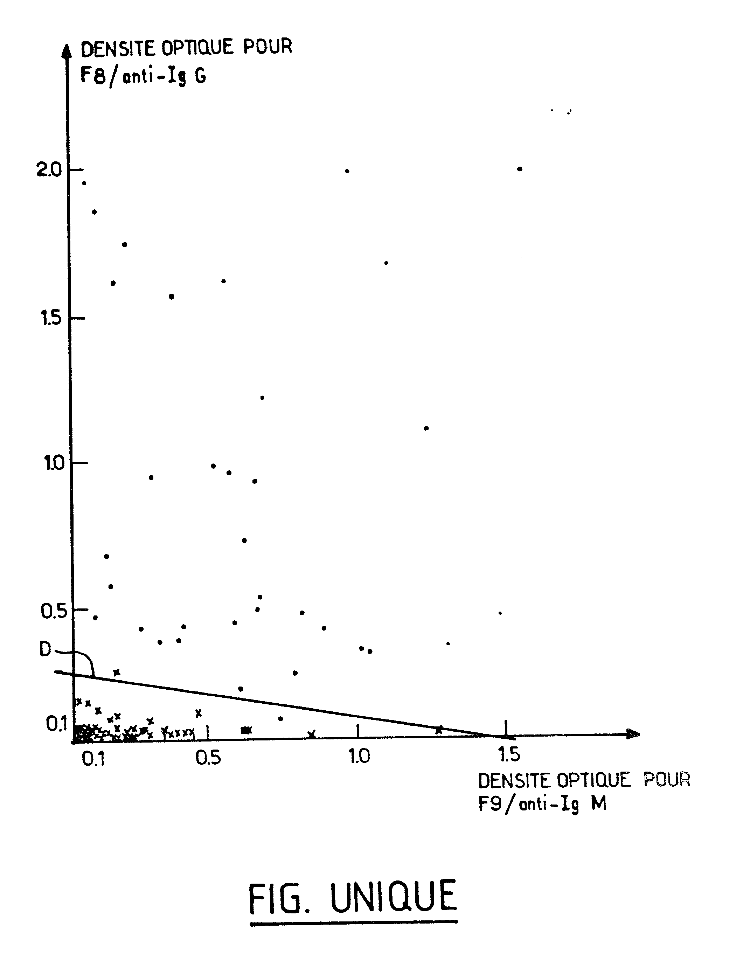

Sur la figure unique, on a représenté un diagramme où chaque sujet soumis au test selon l'invention correspond à un point du diagramme. L'abscisse de ce point est la valeur de la densité optique lue sur le lecteur susmentionné pour un test correspondant à la fraction F9 avec un anti-Ig de type M ; l'ordonnée de ce point correspond à la densité optique obtenue sur le lecteur susmentionné pour une fraction antigénique F8 avec des anti-Ig de type G. On constate que tous les sujets malades ont des points représentatifs disposés au-dessus de la droite D du plan, alors que tous les sujets bien portants ont des points représentatifs au-dessous de ladite droite D (les points correspondant à des sujets-témoins sont représentés par une croix, alors que ceux correspondant à des sujets malades sont représentés par un point rond).

Les mêmes résultats d'essais ont été traités statistiquement de façon différente, tous les témoins étant considérés comme un groupe unique par rapport au groupe des malades. En effectuant les corrélations, on obtient le tableau suivant :

| | Anti-Ig G/F8 | Anti-Ig M/F8 | Anti-Ig G/F9 | Anti-Ig M/F9 |
|---|---|---|---|---|
| Anti-Ig G/F8 | 1 | | | |
| Anti-Ig M/F8 | 0,37 | 1 | | |
| Anti-Ig G/F9 | 0,64 | 0,4 | 1 | |
| Anti-Ig M/F9 | 0,48 | 0,82 | 0,4 | 1 |

EP 0 306 360 A1

On constate que les fractions F8 et F9 associées aux anti-Ig G ou M sont particulièrement intéressantes pour les évaluations globales du risque cardio-vasculaire.

On a pu constater, par ailleurs, que les résultats des mesures opérées par le test selon l'invention avec les fractions antigéniques F5, F7 et F8 d'une part, et l'anti-Ig de type M d'autre part, sont considérablement augmentés au premier jour d'un infarctus et persistent à un niveau élevé pendant au moins un mois. La même constatation a été effectuée avec la fraction antigénique F8 associée à l'anti-Ig de type A. On peut penser que les antigènes correspondant aux fractions F5, F7 et F8 stimulent principalement les immunoglobulines de type M et préexistent avant l'infarctus du myocarde.

On a constaté, de plus, que l'augmentation des anticorps de type M (Ig M) contre les antigènes des fractions F5, F7 et F8 au quatorzième jour et 28ème jour après l'infarctus du myocarde est une partie de la stimulation totale Ig M par l'infarctus.

$$14\text{ème jour : Ig M totale} - F5/\text{Ig M} \quad r = 0,609$$
$$- F7/\text{Ig M} \quad r = 0,702$$
$$- F8/\text{Ig M} \quad r = 0,680$$

$$28\text{ème jour : Ig M totale} - F5/\text{Ig M} \quad r = 0,739$$
$$- F7/\text{Ig M} \quad r = 0,917$$
$$- F8/\text{Ig M} \quad r = 0,711$$

Au vingt-huitième jour après l'infarctus du myocarde, il existe une corrélation linéaire entre le titre d'anticorps "anti-smooth muscle" et les anticorps Ig M contre la fraction antigénique F5 (r = 0,722), la fraction antigénique F7 (r = 0,916) et la fraction antigénique F8 (r = 0,669).

## Revendications

1 - Procédé de préparation de fraction(s) antigénique(s) destinée(s) à la détection des anticorps dont la présence en certaine quantité dans le sérum sanguin d'un sujet est corrélée avec le risque d'accident cardio-vasculaire dudit sujet, caractérisé par le fait que :
- dans une première phase, on réalise un broyat de tissu artériel humain constituant la matière première des fractions recherchées ;
- dans une deuxième phase, on délipide le broyat par traitement au(x) solvant(s) à température ambiante ;
- dans une troisième phase, on reprend le résidu solide de la deuxième phase et on fait agir sur ces constituants, pour obtenir une pluralité de fraction(s) protéinique(s), au moins un agent pris dans le groupe formé par une solution aqueuse d'un sel de métal alcalin ou alcalino-terreux à température ambiante, un acide à chaud, de l'urée, de la guanidine et des enzymes protéolytiques.

2 - Procédé selon la revendication 1, caractérisé par le fait que, dans la troisième phase, on soumet le résidu solide de la deuxième phase au traitement par solution(s) aqueuse(s) de sel de métal alcalin ou alcalino-terreux, on reprend le résidu de cette opération en le soumettant au traitement acide à chaud, puis on soumet tout ou partie du nouveau résidu ainsi obtenu à l'un des autres agents de traitement prévus.

3 - Procédé selon la revendication 2, caractérisé par le fait qu'après le traitement acide à chaud, on fractionne en quatre parties le résidu non solubilisé, la première partie étant soumise à température ambiante à une solution aqueuse d'urée, la deuxième partie étant soumise à température ambiante à une solution aqueuse de guanidine, la troisième partie étant soumise à un traitement à l'élastase entre 30 et 45°C, et la quatrième partie étant soumise à un traitement à la pronase entre 30 et 45°C.

4 - Procédé selon la revendication 3, caractérisé par le fait que l'on réalise les traitements enzymatiques des troisième et quatrième parties du résidu à un pH compris entre 8,3 et 9,2, et qu'on maintient lesdits traitements pendant un temps compris entre 8 et 16 heures, ces traitements étant ensuite arrêtés par chauffage à 60-70°C pendant au moins 30 minutes.

5 - Procédé selon l'une des revendications 3 ou 4, caractérisé par le fait que l'on soumet la première partie du résidu à une solution aqueuse d'urée à un pH compris entre 5,5 et 6,5, à une concentration comprise entre 7 et 9 moles/l, sous agitation, pendant 20 à 30 heures.

6 - Procédé selon l'une des revendications 3 à 5, caractérisé par le fait que l'on soumet la deuxième partie du résidu à une solution aqueuse de guanidine à un pH compris entre 5,5 et 6,5, à une concentration comprise entre 3 et 5 moles/l, sous agitation pendant 20 à 30 heures.

7 - Procédé selon la revendication 1, caractérisé par le fait que, dans la troisième phase, ou soumet le résidu solide de la deuxième phase au traitement par solution(s) aqueuse(s) de sel de métal alcalin ou alcalino-terreux, on reprend les fractions liquides obtenues pour reformer de façon connue des fibres de collagène et l'on traite lesdites fibres reconstituées par l'un des autres agents de traitement prévus pour ladite troisième phase.

8 - Procédé selon selon la revendication 7, caractérisé par le fait que l'on fractionne les fibres reconstituées en cinq parties, les quatre premières parties étant soumises chacune à un des traitements définis dans les revendications 3 à 6, la cinquième partie étant soumise à un traitement acide à chaud.

9 - Procédé selon l'une des revendications 9 ou 8, caractérisé par le fait que le traitement acide à chaud est réalisé au moyen d'une solution aqueuse d'acide trichloracétique à une concentration comprise entre 0,1 et 0,5 mole/l, à une température comprise entre 85 et 105°C pendant un temps compris entre 15 et 45 minutes.

10 - Fractions antigéniques obtenues sous forme de solutions aqueuses dans le procédé selon l'une des revendications 1 à 9.

11 - Utilisation d'au moins une fraction antigénique selon la revendication 10 pour le diagnostic d'un risque cardio-vasculaire, caractérisée par le fait que l'on réalise une électrosynérèse en mettant en oeuvre, dans un support approprié, d'une part, un liquide antigénique contenant au moins une desdites fractions antigéniques et, d'autre part, le sérum sanguin dudit sujet.

12 - Utilisation selon la revendication 11, caractérisée par le fait que le support de l'électrosynérèse est un gel d'agarose ou une bande d'acétate de cellulose, la différence de potentiel entre les zones-réservoirs respectivement occupées par le liquide antigénique et le sérum étant maintenue entre 100 et 200 volts pendant un temps de 60 à 90 minutes.

13 - Utilisation selon l'une des revendications 11 ou 12, caractérisée par le fait qu'après l'électrosynérèse, le support est plongé dans un colorant ayant une affinité pour les protéines, puis dans un décolorant dont l'action ne laisse subsister que la trace colorée des complexes antigènes-anticorps.

14 - Utilisation selon l'une des revendications 11 ou 12, caractérisée par le fait qu'après l'électrosynérèse, le support est traité avec des immunoglobulines animales anti-immunoglobulines humaines (anti-Ig), ces anti-Ig étant marquées à la peroxydase de façon connue, la révélation des anti-Ig fixées par les anticorps du sérum, qui se sont complexés sur les antigènes du liquide antigénique pendant l'électrosynérèse, étant assurée par un mélange (précurseur de coloration -eau oxygénée).

15 - Utilisation selon la revendication 14, caractérisée par le fait que le précurseur de coloration utilisé est l'orthophénylènediamine.

16 - Utilisation d'au moins une fraction antigénique selon la revendication 10 pour le diagnostic d'un risque cardio-vasculaire pour un sujet vivant, caractérisée par le fait que :
- on dispose le liquide antigénique dans un récipient à surface préalablement traitée contenant un milieu tamponné ayant un pH compris entre 8,5 et 9,5 ; on laisse évaporer à sec et on lave le récipient ;
- on sature le récipient avec une solution aqueuse de sérum albumine d'origine animale tamponnée à un pH compris entre 7 et 7,6 pendant 15 à 45 minutes à température ambiante ;
- on dilue le sérum à étudier dans un tampon à pH compris entre 7 et 7,6 dans un rapport compris entre 1/50 et 1/2000 et on le verse dans le récipient en ajoutant du sérum albumine d'origine animale pour saturer les sites non occupés.
- après un temps de contact de 30 à 60 minutes, on lave le récipient à l'eau physiologique et on ajoute des "anti-Ig" d'origine animale marquées, de façon connue, à la peroxydase ;
- après un temps de contact compris entre 30 et 60 minutes, on lave le récipient et on révèle les complexes (antigènes/anticorps/anti-Ig) formés par une solution aqueuse de substrat précurseur de coloration en présence d'eau oxygénée, la révélation étant stoppée après un temps donné ;
- on lit l'intensité de la coloration obtenue en raison de la présence des complexes contenant les anti-Ig marquées et on en déduit l'évaluation du risque cardio-vasculaire.

17 - Utilisation selon la revendication 16, caractérisée par le fait que l'on choisit comme précurseur de coloration l'orthophénylènediamine tamponnée à un pH compris entre 4,8 et 5, 1, l'arrêt de la révélation colorimétrique étant obtenue par addition d'une solution aqueuse d'un acide et d'un réducteur.

FIG. UNIQUE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 492 753 (SHELL et al.) <br> * Résumé * <br> --- | 1 | C 07 K 3/00 <br> C 07 K 15/00 <br> A 61 K 39/00 <br> G 01 N 33/68 <br> G 01 N 33/564 |
| A | US-A-4 343 734 (LIAN et al.) <br> * Résumé * <br> --- | 1 | |
| A | CHEMICAL ABSTRACTS, vol. 75, no. 17, octobre 1971, page 220, résumé no. 107642m, Columbus, Ohio, US; A.M. ROBERT et al.: "Arterial lesions produced in rabbits by immunization with elastin and structural glycoproteins of aorta. Biochemical and morphological studies", & ATHEROSCLEROSIS 1971, (13)3, 427-49 <br> * Résumé * <br> --- | 1 | |
| A | BIOLOGICAL ABSTRACTS, vol. 69, no. 10, 1980, page 6690, résumé no. 62690, Biological Abstracts, Inc., Philadelphia, PA, US; W. HOLLANDER et al.: "Soluble proteins in the human atherosclerotic plaque: With spectral reference to immunoglobulins, complement C-3, alpha1-antitrypsin and alpha2-macroglobulin", & ATHEROSCLEROSIS (34)4: 391-406. 1979 <br> * Résumé * <br> ---      -/- | 1 | DOMAINES TECHNIQUES RECHERCHES (Int. Cl.4) <br><br> G 01 N <br> A 61 K |

Le présent rapport a été établi pour toutes les revendications

| Lieu de la recherche | Date d'achèvement de la recherche | Examinateur |
|---|---|---|
| LA HAYE | 02-12-1988 | OSBORNE H.H. |

Office européen
des brevets

RAPPORT DE RECHERCHE EUROPEENNE

Numero de la demande

EP 88 40 1907

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | BIOLOGICAL ABSTRACTS, vol. 80, no. 11, 1985, page 437, résumé no. 95651, Biological Abstracts, Inc., Philadelphia, PA, US; R. VLAICU et al.: "Quantitative determinations of immunoglogulins and complement components in human aortic atherosclerotic wall", & REV ROUM MED MED INTERNE 23(1): 29-36, 1985 * Résumé * | 1 | |
| A | EP-A-0 189 688 (RESEARCH DEVELOPMENT CORP. OF JAPAN) * Pages 15,16 * | 1 | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 02-12-1988 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)